**Europäisches Patentamt**

**European Patent Office**

**Office européen des brevets**

(19)

(11) Veröffentlichungsnummer : **0 331 040 B1**

(12) **EUROPÄISCHE PATENTSCHRIFT**

(45) Veröffentlichungstag der Patentschrift :
18.12.91 Patentblatt 91/51

(51) Int. Cl.⁵ : **A61M 29/02, F16L 11/08**

(21) Anmeldenummer : **89103281.5**

(22) Anmeldetag : **24.02.89**

(54) **Dilatations-Ballonkatheter.**

(30) Priorität : **28.02.88 DE 3806367**

(43) Veröffentlichungstag der Anmeldung :
**06.09.89 Patentblatt 89/36**

(45) Bekanntmachung des Hinweises auf die
Patenterteilung :
**18.12.91 Patentblatt 91/51**

(84) Benannte Vertragsstaaten :
**AT BE CH DE ES FR GB GR IT LI LU NL SE**

(56) Entgegenhaltungen :
**EP-A- 0 186 267**
**WO-A-87/00442**
**DE-A- 25 441**
**DE-A- 3 337 258**
**FR-A- 2 254 746**

(73) Patentinhaber : **DEUTSCHE INSTITUTE FÜR
TEXTIL- UND FASERFORSCHUNG
STUTTGART Stiftung des öffentlichen Rechts
Körschtalstrasse 26
W-7306 Denkendorf (DE)**

(72) Erfinder : **Planck, Heinrich, Dr.-Ing.
Weinbergstrasse 66
W-7440 Nürtingen 10 (DE)**

(74) Vertreter : **Gleiss, Alf-Olav, Dipl.-Ing. et al
Patentanwaltskanzlei Gleiss & Grosse
Silberburgstrasse 187
W-7000 Stuttgart 1 (DE)**

## Beschreibung

Die Erfindung betrifft einen Dilatations-Ballonkatheter — nachfolgend kurz Ballonkatheter genannt — gemäß dem Oberbegriff des Anspruches 1.

Nachfolgend werden folgende Definitionen gebracht:

Unter "fluidumsdicht" ist Undurchlässigkeit für Gase, Dämpfe und Flüssigkeiten verstanden. Unter einem Fluid ist ein Gas, Dampf oder eine Flüssigkeit verstanden. Unter einem Monofilament (oft auch Monofilamentgarn, Monofilgarn, Monofil oder Endlosfaser genannt) ist ein Faden verstanden, der aus einem einzigen Filament besteht, gleichgültig, wie groß sein Durchmesser ist. Unter einem Multifilament (auch Multifilamentgarn, Multifilgarn genannt), ist ein Faden verstanden, der aus einer Mehrzahl von Filamenten besteht. Die Filamente können vorzugsweise endlose Chemiefasern sein.

Ballonkatheter, auf die sich die Erfindung bezieht, können so ausgebildet sein, daß die Katheterleitung an ihrem einen Ende fluidumsdicht abgeschlossen ist. Die Erfindung bezieht sich jedoch auch auf Ballonkatheter, bei denen die Katheterleitung mindestens ein Lumen aufweist, das sich über die volle Länge der Katheterleitung erstreckt und an beidem axialen Enden offen ist. Allgemein bezieht sich die Erfindung auf Ballonkatheter, die von außen in die Körper von Lebewesen, insbesondere von Menschen, durch natürliche oder künstliche Öffnungen eingeführt werden. In eingeführtem Zustand kann dann der Ballon durch in ihn eingeleitetes Druckfluid, das vorzugsweise Gas oder eine Flüssigkeit sein kann, aufgeweitet werden, um sich in der betreffenden Körperöffnung, einem Harnleiter, einer sonstigen Leitung oder Kanal, einem Gefäß, einer Operationswunde oder dgl. festzuhalten, und/oder in dieser Körperöffnung oder dgl. einen Verschluß zu bilden oder die Körperöffnung oder dgl. aufzuweiten.

Erfindungsgemäße Ballonkatheter können unterschiedliche medizinische Anwendungszwecke haben, bspw. für Zwecke der Nephrostomie, zum Einführen in Blutgefäße, Harnleiter, Operationswunden oder sonstigen beliebigen medizinschen Zwecken dienen.

Bei einem bekannten Ballonkatheter dieser Art (DE-OS 3337258) weist ein Schaft des Katheters ein Rohr aus geflochtenem Material auf, das über einen größeren Bereich seiner Länge in einen inelastischen Mantel aus halbstarrem Material eingekapselt ist, der sich von dem proximalen Ende des Katheters über einen größeren Abschnitt der Länge des Schaftes erstreckt. Ein äußerer Mantel aus elastischem Material erstreckt sich über einen kleineren Abschnitt der Länge des Schaftes am oder im Bereich des distalen Endes des Katheters, wodurch dieser kleinere Abschnitt des Schaftes als Ballon aufblasbar bzw. erweiterbar ist. Hierdurch soll sich der Ballon nicht über akzeptable Grenzen hinaus aufweiten lassen.

Ebenso sind Dilatations-Ballonkatheter bekannt, in welchen bei dem Katheter die gleiche zweilumige Konstruktion vorliegt, wie oben beschrieben, jedoch ist hier ein äußerer Mantel aus unelastischem, durch Strahlenvernetzung erzeugtem Polyethylen (PE) vorhanden. Hier wird solange Fluid in den Ballon gedrückt, bis der PE-Mantel, der vorher in ungefülltem Zustand um den Katheter herum gefaltet vorgelegen hat, bis zu seiner maximalen Kontur entfaltet wurde. Während dieser Entfaltung erhöht sich der Innendruck nur unwesentlich, erst am Ende des Entfaltungsvorgangs, wenn die maximale Größe des Ballons erreicht ist, steigt der Druck deutlich an. Die Begrenzung der Endkontur geschieht durch die Abmessung der kompletten PE-Ballonmanschette. Auch gibt es Dilatations-Ballonkatheter, in welchen der äußere Mantel aus elastischen Fäden mit elastischem Überzug besteht.

Der Aufblasvorgang bei dieser Art von Katheter verläuft von Beginn an mit einem Anstieg des Druckwertes; gegen Ende, wenn die maximale Kontur erreicht ist, hat auch der Druckwert sein Maximum erreicht. Weitere Steigerung des Druckes führt zum Bersten des Ballons. Die Konturbegrenzung wird dabei durch die Elastizitätswerte der in die Wand des Ballons eingeflochtenen Fäden erzeugt. Aufgrund der Flechtcharakteristik verbiegen sich jedoch diese Ballons in unerwünschte Formen, beispielsweise in Bananenform, wenn der maximale Aufblasdruck erreicht ist. Die Handhabung, z.B. das Überschieben eines Rohres, wie es bei Nephrostomie-Ballonkathetern nötig ist, über den Ballon wird durch die "falsche" Form sehr stark erschwert oder verhindert.

Die Erfindung bezweckt die Schaffung eines Ballonkatheters, dessen Ballon sich nicht beliebig aufweiten lassen soll, darüber hinaus aber innenseitig mit besonders hohen Drücken (Dilatationsdrücke) des Druckfluids beaufschlagt werden kann, um so dem Aufweiten des in Körperöffnungen, Blutgefäßen, Harnleitern, Harnröhren usw. eingesetzten Ballons des Ballonkatheters ggf. örtlich Widerstand entgegensetzende Sehnen, Fascien oder sonstige Widerstände durch hohe Dilatationsdrücke des Ballons wegschieben zu können. Auch soll die Erfindung es ermöglichen, den Ballon lang auszubilden und dennoch erzielen zu lassen, daß der Ballon auch in aufgeweitetem Zustand noch schlauchähnliche Gestalt aufweist, vorzugsweise im wesentlichen konstanten Außendurchmesser seines aufgeweiteten Bereiches.

Diese Aufgabe wird erfindungsgemäß durch einen Ballonkatheter gemäß Anspruch 1 gelöst.

Der Ballon des erfindungsgemäßen Ballonkatheters läßt sich infolge der Stränge aus Hochmodulfasern (englisch: high modulus fibres) trotz der elastomeren Fäden der beiden Fadenscharen auch mit hohen Drücken nicht stark aufweiten, ergibt jedoch auch bei großer Länge des Ballons, daß der Außendurchmesser des aufgeweiteten Ballonbereiches über dessen Länge im wesentlichen konstant ist, also in gerader Lage ungefähr kreiszylindrische Gestalt hat. Es gelingt sogar, daß der aufgeweitete Ballon, von seinen axialen Enden abgesehen, praktisch streng zylindrische, insbesondere kreiszylindrische Gestalt aufweist, also hier überall den gleichen kreisförmigen Querschnitt aufweist. Hochmodulfasern sind bekannt und bedürfen deshalb keiner näheren Erläuterung. Sie zeichnen sich durch geringe Reißdehnung von maximal einigen Prozenten und hohe Festigkeit aus. Die Hochmodulfasern können bspw. zweckmäßig aus hochmolekularem Polyethylen und/oder Para-Aramid und/oder Kohlenstoff und/oder anderen geeigneten hochfesten Materialien bestehen. Hochmodulfasern aus Para-Aramid sind z.B. unter dem Handelsnamen "Kevlar" bekannt. Die elastomeren Fäden der beiden miteinander verflochtenen Fadenscharen sind im weiteren oft auch als Flechtfäden bezeichnet. Sie bestehen aus elastomeren Fasern. Diese Flechtfäden können vorzugsweise Monofilamente oder Multifilamente sein oder gewünschtenfalls aus Spinnfasern hergestellt sein. Diese Flechtfäden können bspw. aus Kunststoff, vorzugsweise segmentiertem Polyurethan, oder aus Gummi, Kautschuk, Latex oder sonstigen geeigneten elastomeren Materialien bestehen. Bspw. können ihre Fasern solche sein, die unter dem Handelsnamen "Lycra" (Hersteller Du Pont) im Handel erhältlich sind.

Die Fäden der Stränge werden nicht als Flechtfäden bezeichnet, sondern, falls erforderlich, zur Unterscheidung von den Flechtfäden als Strangfäden.Auch die Strangfäden können vorzugsweise Monofilamente oder Multifilamente sein. Es ist jedoch auch möglich, daß die Strangfäden aus Spinnfasern hergestellt sind, wenn auch in diesem Falle ausreichende Festigkeit bei geringer Reißdehnung vorliegt.

Die Reißdehnung der Fäden der Strangfäden kann vorzugsweise maximal 10%, besonders zweckmäßig maximal 6% betragen, besonders vorteilhaft sind Reißdehnungen von mindestens 1%, vorzugsweise von minimal 2% und/oder maximal 5%. Die Stränge verhindern selbst dann zu starkes Aufweiten des Ballons, wenn versehentlich das dem Aufweiten dienende Druckfluid mit etwas zu großen Drücken in den Ballon eingeleitet wird, wodurch eventuelle Bedienungsfehler keine negativen Auswirkungen haben.

Auch verhindern die Stränge mit Sicherheit ein Überdehnen der elastomeren Flechtfäden, so daß diese ihre Elastizität auch nach langer Betriebszeit noch haben und so der Ballon auch nach langer Betriebszeit des Ballonkatheters nach jedesmaligem Gebrauch wieder seine ursprüngliche Gestalt annimmt und so dieser Ballonkatheter im Betrieb keine störende Vergrößerung des unaufgeweiteten Ballons erfährt.

Die Erfindung ergibt auch den wichtigen Vorteil, daß der Ballon mit Druckfluid hohen Druckes aufgeweitet werden kann, bspw. mit Drücken von mehreren bis vielen bar, bspw. mit Drücken von 2 bis 20 bar, so daß der Ballon bei seinem Aufweiten bspw. Knorpel, Fascien, Sehnen, Muskelschichten und sonstige Widerstände infolge des hohen Balloninnendruckes beiseite schieben kann, was einen geradlinigen, besseren Sitz des Ballonkatheters in dem betreffenden Gefäß, Öffnung, Kanal oder dgl. des betreffenden Lebewesens und auch eine besonders gute Abdichtung umfangsseitig des Ballons ergibt bzw. ermöglicht.

Die Länge des Ballons kann je nach gewünschtem Anwendungszweck unterschiedlich sein, bspw. zweckmäßig 10-400 mm betragen, besonders zweckmäßig zirka 40-300 mm.

Dabei kann die Wandstärke des Ballons relativ gering sein, bspw. 0,5-1,2 mm oder je nach Fall auch noch weniger oder mehr betragen. Die Katheterleitung, also der Katheterschlauch oder ein starres Katheterrohr können ebenfalls geringe Außendurchmesser aufweisen von bspw. von 0,8-3 mm oder je nach Erfordernis auch noch weniger oder mehr. Die Katheterleitung kann vorzugsweise ungefähr konstantes Profil über ihre Länge aufweisen, vorzugsweise kreisringförmiges Außenprofil.

Besonders zweckmäßig u.a. für leichtes Einführen des Ballon katheters in Körperöffnungen und dgl. ist es, wenn der Ballon im entleerten, also im nicht aufgeweiteten Zustand, ohne Falten zu werfen, an der Katheterleitung vorzugsweise dicht anliegt, besonders zweckmäßig am gesamten von ihm umfaßten Umfang der Katheterleitung.

Der Ballon muß nach außen fluidumsdicht abgedichtet sein. Hierzu kann der Flechtschlauch auf seiner Außenseite und/oder Innenseite einen fluidumsdichten dehnbaren schlauchförmigen Mantel aufweisen. In vielen Fällen kann vorgesehen sein, nur innenseitig oder nur außenseitig des Flechtschlauches einen solchen Mantel vorzusehen. Vorzugsweise kann sowohl innenseitig als auch außenseitig des Flechtschlauches je ein Mantel vorgesehen sein. Der Mantel kann bspw. aus elastomerem Material bestehen, bspw. aus Kunststoff, Gummi oder dgl. Wenn zwei Mäntel vorgesehen sind, können die beiden Mäntel vorzugsweise durch Durchbrechungen des Flechtschlauches durchdringende Klebstellen oder Schweißstellen miteinander nichtlösbar verbunden sein.

Der Innenmantel und/oder Außenmantel des Flechtschlauches kann mit diesem vorzugsweise verklebt sein. Die Verklebung kann adhäsiv durch Einbringung zusätzlichen Klebstoffs oder auch kohäsiv durch Anlösen des oder der betreffenden Mäntel erfolgen.

Die elastische Dehnung des Mantels oder der Mäntel kann vorzugsweise so groß sein, daß sie im Betrieb nicht überschritten wird.

Die Länge des aufweitbaren Bereichs des Ballons beträgt mindestens das 4-fache des Außendurchmessers dieses Ballonbereiches in unaufgeweitetem Zustand, um so trotz der geringen Reißdehnung der Stränge genügende Aufweitbarkeit des Ballons zu erreichen. Vorzugsweise kann diese Länge des aufweitbaren Bereichs des Ballons mindestens das 10-fache, vorzugsweise mindestens das 15-fache des maximalen Außendurchmessers dieses Ballonbereichs in unaufgeweitetem Zustand betragen.

Es kann bevorzugt vorgesehen sein, daß der Flechtschlauch so ausgebildet ist, daß der lichte Innendurchmesser des Ballons, wenn er durch Druckfluid so weit aufgeweitet ist, daß die Stränge auf zirka 90% ihrer Reißdehnung beansprucht sind, im aufgeweiteten Bereich maximal das 5-fache, vorzugsweise maximal das 2-fache, insbesondere oft zweckmäßig maximal das 1,5-fache des lichten Innendurchmessers des nicht aufgeweiteten Ballons beträgt.

Der Flechtschlauch kann vorzugsweise so ausgebildet sein, daß benachbarte Stränge so verlegt sind, daß der eine Strang die Fäden der ersten Fadenschar von außen gesehen überläuft und die Fäden der zweiten Fadenschar unterläuft, wogegen der andere Strang die Fäden der ersten Fadenschar unterläuft und die Fäden der zweiten Fadenschar überläuft. Es kommen jedoch auch andere Verlegungen der Stränge oft zweckmäßig infrage. So kann oft zweckmäßig vorgesehen sein daß die Stränge des Flechtschlauches so verlegt sind, daß übernächste Gruppen aus n zueinander benachbarten Strängen die Fäden der ersten Fadenschar von außen gesehen überlaufen und die Fäden der zweiten Fadenschar unterlaufen und daß die dazwischen liegenden Stranggruppen, von denen jede aus je m Strängen besteht, die Fäden der ersten Fadenschar unterlaufen und die Fäden der zweiten Fadenschar überlaufen, wobei n und m ganze Zahlen größer 1 sind und n und m für beide Stranggruppen vorzugsweise gleich groß sind. Bevorzugt können n und m gleich 2 oder 3 sein.

Ferner kann zweckmäßig vorgesehen sein, daß mindestens ein Strang, vorzugsweise alle Stränge aus je einem einzigen Faden bestehen. Es ist jedoch auch möglich und ebenfalls oft zweckmäßig, vorzusehen, daß mindestens ein Strang, vorzugsweise alle Stränge aus je einem mehrere Fäden aufweisenden Fadenbündel bestehen. Die Fäden der Stränge können zweckmäßig sogenannte glatte Fäden sein, die also nicht texturiert sind. Die Flechtfäden der beiden Fadenscharen können ebenfalls zweckmäßig glatte, also nicht texturierte Fäden sein.

Jeder Faden einer Fadenschar kann zweckmäßig so verlegt sein, daß er, von außen gesehen, die von ihm gekreuzten Fäden der anderen Fadenschar abwechselnd überläuft und unterläuft.

Die Zahl der Stränge des Flechtschlauches kann zweckmäßig maximal der Zahl der Klöppel entsprechen, von denen die Flechtfäden der beiden Fadenscharen beim Flechten des Flechtschlauches abliefen. Bevorzugt kann die Zahl der Stränge der Hälfte dieser Klöppelzahl entsprechen, oder auch größer oder kleiner sein.

Die Befestigung des Ballons auf der Katheterleitung kann in irgendeiner geeigneten Weise vorgesehen sein, vorzugsweise gemäß den Maßnahmen nach Anspruch 14 oder 15.

In der Zeichnung sind Ausführungsbeispiele der Erfindung dargestellt. Es zeigen :

Fig. 1     eine teilweise geschnittene und gebrochene Seitenansicht eines Ballonkatheters gemäß einem Ausführungsbeispiel der Erfindung,

Fig. 2     einen vergrößerten, gebrochenen Längsschnitt durch den Ballonkatheter gemäß Fig. 1,

Fig. 3     einen Schnitt durch den Ballonkatheter nach Fig. 2, gesehen entlang der Schnittlinie 3-3,

Fig. 4     eine Darstellung eines Flechtschlauches zur Erläuterung seines Aufbaus, gesehen in Richtung seiner Längsachse, wobei die übrigen Teile des Ballonkatheters in diesem Ausführungsbeispiel strichpunktiert angedeutet sind,

Fig. 5     eine ausschnittsweise Draufsicht auf eine Abwicklung des Flechtschlauches nach Fig. 4 zur Darstellung seiner Struktur,

Fig. 6     eine Variante des Flechtschlauches nach Fig. 4, gesehen ebenfalls in dessen axialer Richtung.

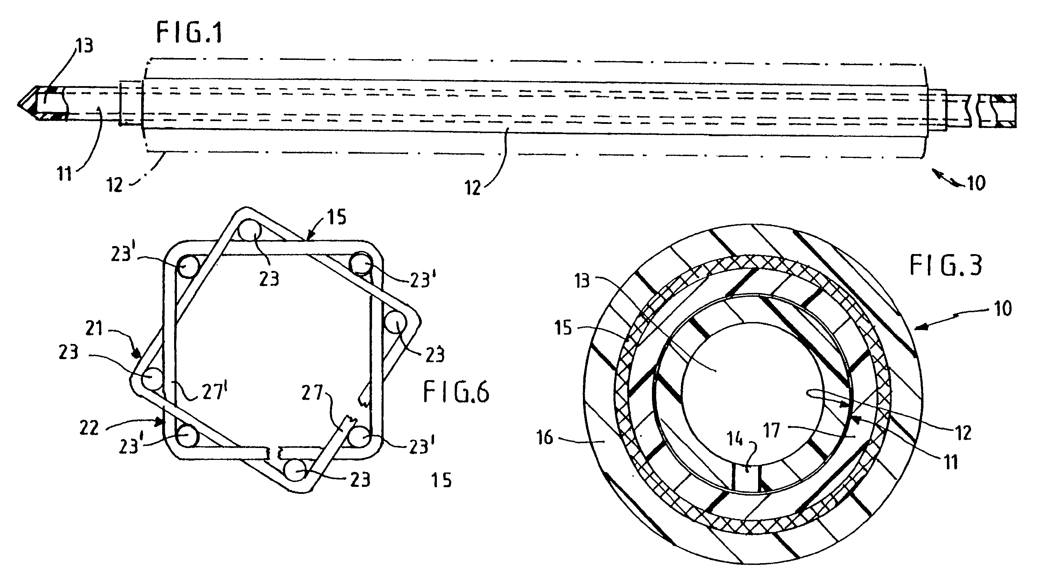

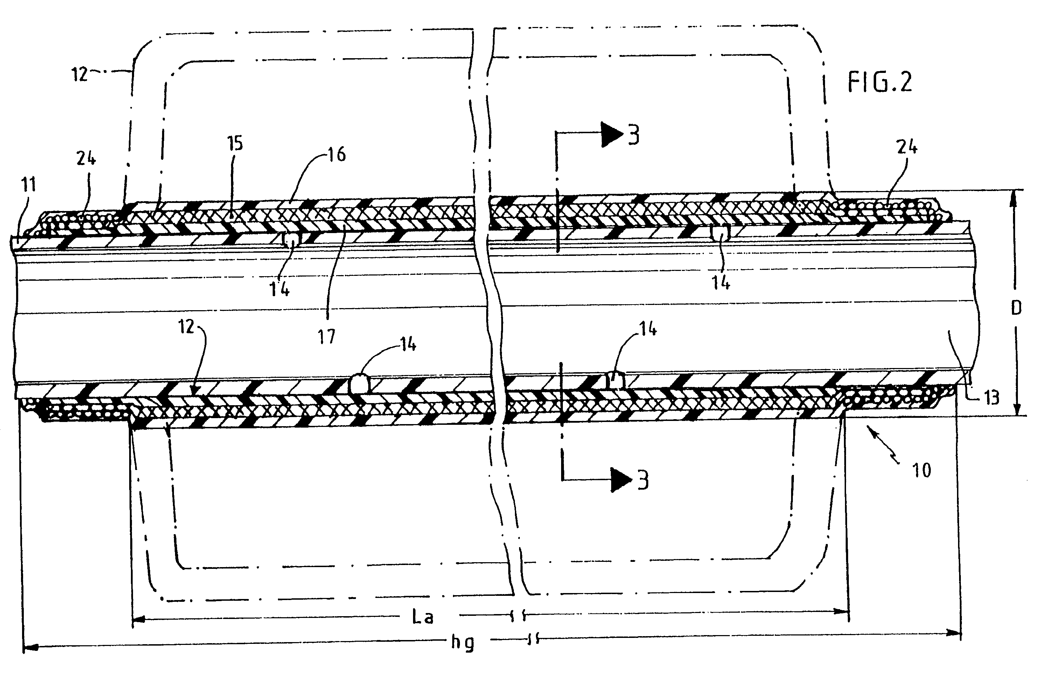

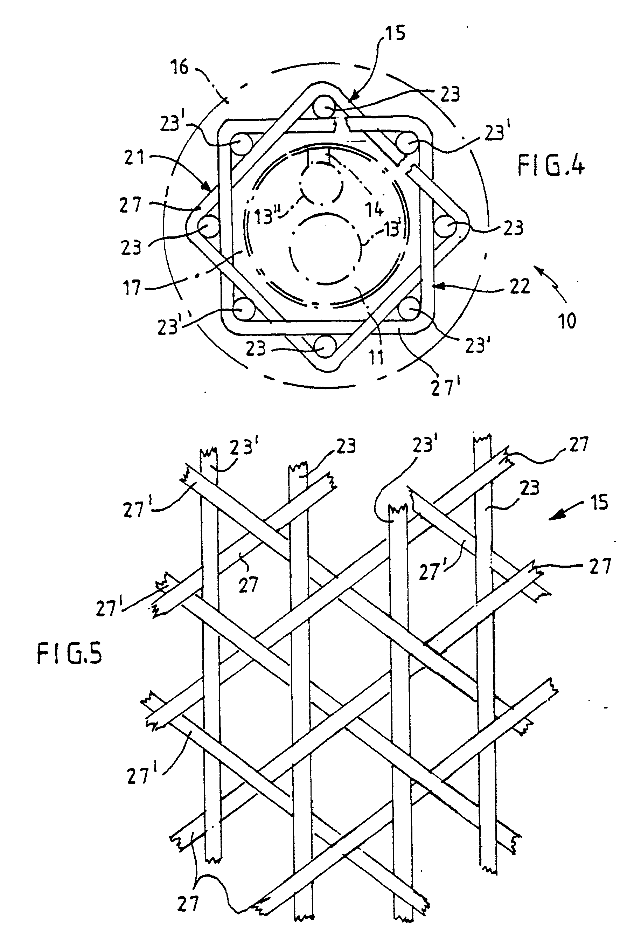

Der Ballonkatheter 10 gemäß den Figuren 1 und 2 weist eine Katheterleitung 11 und einen auf ihrem linksseitigen Endbereich angeordneten, aufweitbaren Ballon 12 auf, der keinerlei Falten aufweist. Die Katheterleitung 11 ist als dünner, am linksseitigen Stirnende geschlossener, flexibler Schlauch konstanten kreisrunden Querschnittprofiles ausgebildet und besteht hier aus Kunststoff, bspw. Polyurethan, kann jedoch auch aus anderen geeigneten flexiblen oder starren Materialen bestehen, die durch das dem jeweiligen Aufweiten des Ballons 12 dienende, in das Lumen 13 dieses Schlauches, d.h. in diesem Ausführungsbeispiel in seinen einzigen durchgehenden Kanal eingeleitete Druckfluid, (z.B. Luft oder Flüssigkeit) nicht störend aufgeweitet werden. Dieses Druckfluid kann dank der nachfolgend beschriebenen Ausbildung des Ballons 12 sehr hohen Dilatationsdruck, d.h. Druck zum Aufweiten des Ballons aufweisen von bspw. 6 bis 20 bar. Auch hat der Ballon 12 die Eigenschaft, daß er sich bereits bei relativ geringen Drücken (Dilatationsdrücken) des Druckfluids schon fast maximal aufweitet und bei weiterer Drucksteigerung dann sich fast nicht mehr weiter aufweitet, jedoch dann durch die höheren Drücke Muskeln, Sehnen und sonstige ggf. störenden Gewebeteile oder dergl. wegschieben

4

kann, die seinem Aufweiten örtlich Widerstand entgegensetzen, so daß man es durch Anwendung hoher Drücke in der Hand hat, dieses Weg schieben zu erreichen oder durch Anwendung niedrigerer Drücke es zu unterlassen.

Wenn die Katheterleitung, wie dargestellt, an ihrem einen Ende abgeschlossen ist, genügt es, wenn sie ein einziges Lumen 13 aufweist, wie es in diesem Ausführungsbeispiel der Fall ist und in das Lumen wird dann das Druckfluid zum Aufweiten des Ballons 12 eingeleitet, wozu in der Wandung des Lumens 13 gegenüber dem aufweitbaren Bereich des Ballons 12 Löcher 14 vorgesehen sind.

Der Ballon 12 liegt in nicht aufgeweitetem Zustand auch mit seinem aufweitbaren Bereich an der Katheterleitung dicht an, und zwar besonders zweckmäßig am gesamten von ihm umfaßten Umfang der Katheterleitung.

Der Ballon 12 weist einen Flechtschlauch 15 auf, der außenseitig und innenseitig mit je einem fluidumsdichten, elastisch dehnfähigen Mantel 16,17 überzogen ist, vorzugsweise mit elastomeren Mänteln 16,17, die untereinander durch Brücken, die Durchbrüche des Flechtschlauches 15 durchdringen, verklebt oder verschweißt sind. Der durch in den Ballon 12 durch dessen Lumen 13 und die Löcher 14 hindurch eingeleitetes Druckfluid aufgeweitete Zustand des Ballons 12 ist strichpunktiert dargestellt und man sieht, daß der aufgeweitete Bereich des Ballons 12 im wesentlichen kreiszylindrische Gestalt aufweist. Durch Ablassen des Druckfluids kehrt der Ballon 12 wieder vollständig in seine ursprüngliche, voll ausgezogen dargestellte Gestalt zurück, auch nach langer Betriebszeit, in der er wieder auf voller Länge an der Katheterleitung 11 faltenfrei anliegt. Auch in aufgeweitetem Zustand hat der Ballon 12 keine Falten.

Ein möglicher und vorteilhafter Aufbau des Flechtschlauches 15 wird anhand eines in den Fig. 4 und 5 dargestellten Ausführungsbeispiels nun näher erläutert, wobei in Fig. 4 die Katheterleitung 11 abweichend von Fig. 1 bis 3 zwei Lumen 13', 13" aufweist, von denen das Lumen 13' das Hauptlumen darstellt, das nicht mit dem Ballon 12 in Verbindung steht, so daß es an beiden Enden der Katheterleitung 11 offen sein kann, wogegen das Lumen 13" nun an einem Ende der Katheterleitung 11 offen ist. Dieses Lumen 13" steht über mindestens ein Loch 14 mit dem Innenraum des Ballons 12 in fluidleitender Verbindung.

Der in den Fig. 4 und 5 ausschnittsweise schematisch dargestellte Flechtschlauch 15 ist aus zwei gegenläufigen Fadenscharen 21, 22 mit je acht Flechtfäden 27 bzw. 27' unter Einsatz von je acht Klöppeln unter Einflechten von acht in axialer Richtung des Flechtschlauches 15 verlaufenden, aus Hochmodulfasern, bspw. zweckmäßig aus Polyäthylen oder Kohlenstoff bestehenden Strängen 23, 23' geflochten. Die elastomeren Flechtfäden der Fadenschar 21 sind also mit 27 und die der Fadenschar 22 mit 27' bezeichnet, wobei nur einige dieser Flechtfäden 27, 27' ausschnittsweise dargestellt sind.

Jeder Strang 23 bzw. 23' besteht in diesem Ausführungsbeispiel aus je einem Monofilament oder Multifilament, vorzugsweise einem glatten Monofilament bzw. aus glatten Filamenten bestehendem Multifilament. Dieser Flechtschlauch weist in diesem Ausführungsbeispiel insgesamt acht Stränge 23, 23' auf. Dann ist es besonders zweckmäßig, wenn die Flechtfäden 27, 27' beim Flechten von je acht Klöppeln kamen, hier also, wenn von jedem Klöppel ein einziger Flechtfaden 27 bzw. 27' ablief, jede Fadenschar 21, 22 aus je acht Flechtfäden besteht, obwohl bspw. die Anzahl der Stränge 23, 23' auch grösser oder kleiner sein kann, vorzugsweise maximal der Anzahl der Klöppel entsprechen kann, von denen die Flechtfäden 27, 27' beim Flechten des Flechtschlauches abliefen. Gewünschtenfalls kann sie jedoch auch größer oder kleiner sein, bspw. einem Viertel der Klöppelanzahl entsprechen. Die Klöppelzahl kann zweckmäßig um so größer sein, je größer der maximale Außendurchmesser des aufgeblasenen Ballons und je größer sein Dilatationsdruck ist.

Die Stränge 23, 23' gehen in diesem besonders vorteilhaften Ausführungsbeispiel zwischen den Flechtfäden 27, 27' wie dargestellt hindurch, derart, daß hier der in Umfangsrichtung des Flechtschlauches gesehen jeweils übernächste Strang 23 die Flechtfäden 27 der Fadenschar 21 unterläuft und die der Fadenschar 22 überläuft. Die dazwischen liegenden Stränge 23' unterlaufen die Flechtfäden 27' der Fadenschar 22 und überlaufen die Flechtfäden 27 der Fadenschar 21. Gemäß Fig. 5 sind die Stränge 23, 23', bezogen auf die zu ihnen parallele Längsachse des geflochtenen Schlauches 15 in gleich großen Zentriwinkeln um den Umfang des Flechtschlauches 15 verteilt, doch können sie bspw. gemäß Fig. 6 auch in ungleich großen Zentriwinkelabständen voneinander angeordnet sein.

Jeder Strang 23, 23' erstreckt sich also in axialer Richtung des Flechtschlauches. Die Stränge 23, 23' sind parallel zueinander und verlaufen nicht wendel oder spiralförmig um den Schlauch herum, sondern jeder Strang verläuft bei geradem Ballon in einer Längsmittelebene des Flechtschlauches 15.

Es sei nunmehr ein zahlenmäßiges Ausführungsbeispiel des Flechtschlauches 15 gebracht:

Wandstärke des Ballons 12 zirka 0,9 mm. Außendurchmesser der Katheterleitung 11 zirka 2,3 mm. Maximaler Außendurchmesser D des aufweitbaren Bereiches des unaufgeweiteten Ballons zirka 4,1 mm. Gesamtlänge hg des Ballons 12 zirka 200 mm. Länge La des aufweitbaren Bereiches des Ballons 12 zirka 170 mm. Die Flechtfäden 27, 27' sind Multifilamentfäden, die aus Filamenten aus Gummi, bspw. aus Lycra (Lederer) bestehen. Die Feinheit des einzelnen Flechtfadens 27, 27' beträgt zirka 406 dtex. Ihr Zug-Elastizitätsmodul $E_{100}$

betrug 0,046 N/mm². Jeder Strang 23 und 23' ist ein Multifilament aus Para-Aramid einer Feinheit von 380 den. Die Stränge 23, 23' sind ebenso wie die Flechtfäden 27, 27' mit den Mänteln 16, 17 verklebt und miteinander durch die Durchbrechungen des Flechtschlauches 15 durchdringende Brücken verklebt oder verschweißt.

Der Ballon 12 bildet, bevor er auf die Katheterleitung 11 aufgeschoben und auf ihr axial unverrückbar befestigt wird, in geradem Zustand einen kreiszylindrischen Schlauch.

Der Flechtschlauch ist im Ausführungsbeispiel nach Fig. 1 und 2 auf der Katheterleitung 11 dadurch befestigt, in dem er an kurzen axialen Längsendbereichen mit je einem Faden (Umwickelfaden) 24 straff umwickelt ist. Dieser Umwickelfaden reicht noch etwas über die beiden Längsenden des Flechtschlauches 15 und des Innenmantels 17 hinaus auf die Katheterleitung 11. Erst danach ist der Außenmantel 16 aufgebracht worden. Er kann bspw. dadurch aufgebracht sein, daß man den den Ballon 12 aufweisenden Bereich des Ballonkatheters 10 in ein Bad aus einer Polymerlösung eintaucht, so daß sich hierdurch der äußere Mantel 16 als äußerer Überzug oder Schlauch des Ballons 12 und ggf. noch über den Flechtschlauch 15 und den schlauchförmigen Innenmantel hinaus ergibt, der auch die Umwickelfäden 24 vollständig umhüllt, so daß sie sich nicht mehr lösen können. Bei dieser Herstellung verklebt oder verschweißt der Außenmantel 16 von selbst mit dem Innenmantel 17 und verklebt auch mit dem Flechtschlauch 15, wenn dessen Fäden 27, 27', 23, 23' aus mit dem Material des Außenschlauches verklebbaren Material bestehen, was besonders zweckmäßig ist. Nach Herausnehmen des Katheterschlauches mit dem Ballon aus der genannten Polymerlösung trocknet dann der Außenmantel 16. Die Bereiche der Katheterleitung 11, die keinen Außenmantel tragen sollen, werden, soweit möglich, in die Polymerlösung nicht eingetaucht oder man kann den Außenmantel nachträglich von dem oder den betreffenden Bereichen des Katheterleitung 11 vor dem Eintauchen in das Bad mit einer Schutzschicht überziehen, bspw. mit Wachs, die leichtes Entfernen des Außenmantels auf diesen Bereichen ermöglicht.

Der Ballonkatheter kann unterschiedlichen Zwecken dienen, bspw. als Stichkanal-Dilatator, Nephrostomie-Dilatator, Ureter-Dilatator, Urethra-Dilatator, Gefäß-Stenosen-Dilatator und auch noch andere Einsatzgebiete haben.

Wenn man den Ballon 12 des Ballonkatheters 10 durch Einleiten von Druckfluid aufweitet, dann zeigt sich unter anderem auch folgende hervorragende Eigenschaft. Der aufweitbare Ballonbereich behält beim Aufweiten praktisch kreiszylindrische Gestalt und bei einem bestimmten, noch relativ geringen Druck des Druckfluids ist der maximale Außendurchmesser des Ballons praktisch schon erreicht und bei weiterer Erhöhung des Drucks des Druckfluids ändert sich dieser maximale Außendurchmesser praktisch nicht mehr. Der Druck kann dann so weit gesteigert werden, bis er nahe dem Berstdruck ist, bei dem also der Flechtschlauch unter Reißen mindestens eines Stranges bersten würde. Wenn bspw. der Berstdruck 24 bar beträgt, kann man den Druck des Druckfluids bspw. etwa maximal 12 bar vorsehen, um eine voll ausreichende Sicherheitsgrenze vor Erreichen des Berstdrucks zu haben.

Der Ballonkatheter zeichnet sich auch dadurch aus, daß der Ballon 12 nach jedem Aufweiten, wenn der Druck des Druckfluids wieder abgebaut wird, in seine ursprüngliche Gestalt zurückkehrt, in der er an der Umfangswandung der Katheterleitung wieder dicht anliegt. Dies schafft also gleichbleibende Einführungseigenschaften des Ballonkatheters in Stichkanäle, Körperöffnungen, Operationswunden usw., auch nach vielmaligem Gebrauch.

### Patentansprüche

1. Dilatations-Ballonkatheter (10) mit einer länglichen, hohlen Katheterleitung (11), auf der ein durch Druckfluid aufweitbarer, flexibler Ballon (12) axial unverrückbar angeordnet ist, welches Druckfluid durch mindestens ein Lumen (13) der Katheterleitung (11) in den Zwischenraum zwischen dem Ballon (12) und der Katheterleitung (11) einleitbar und aus ihm wieder ableitbar ist, welcher Ballon (12) einen Flechtschlauch (15) aufweist, der mit mindestens einem fluidumsdichten elastischen Mantel (16, 17) versehen ist, dadurch gekennzeichnet, daß der Flechtschlauch (15) aus zwei gegenläufigen Fadenscharen (21, 22) aus elastomeren Fäden geflochten und in dieses Geflecht bei dessen Flechten in Axialrichtung des Flechtschlauches (15) verlaufende Stränge (23, 23') aus Hochmodulfasern so eingeflochten sind, daß der einzelne Strang von außen gesehen über den Fäden der einen Fadenschar (21) und unter den Fäden der anderen Fadenschar (22) verläuft und daß die Länge des aufweitbaren Bereiches des Ballons (12) mindestens das 4-fache des maximalen Außendurchmessers dieses Ballonbereiches in unaufgeweitetem Zustand beträgt.

2. Dilatations-Ballonkatheter nach Anspruch 1, dadurch gekennzeichnet, daß benachbarte Stränge (23, 23') des Flechtschlauches so verlegt sind, daß der eine Strang (23) die Fäden der ersten Fadenschar (21) von außen gesehen überläuft und die Fäden der zweiten Fadenschar (22) unterläuft, wogegen der andere Strang (23') die Fäden der ersten Fadenschar (21) unterläuft und die der zweiten Fadenschar (22) überläuft.

3. Dilatations-Ballonkatheter nach Anspruch 1, dadurch gekennzeichnet, daß die Stränge des Flecht-

EP 0 331 040 B1

schlauches so verlegt sind, daß übernächste Gruppen aus n zueinander benachbarten Strängen die Fäden der ersten Fadenschar von außen gesehen überlaufen und die Fäden der zweiten Fadenschar unterlaufen und daß die dazwischen liegenden Stranggruppen, von denen jede aus je m Strängen besteht, die Fäden der ersten Fadenschar unterlaufen und die Fäden der zweiten Fadenschar überlaufen, wobei n und m je eine ganze Zahl größer 1 sind und n und m für beide Stranggruppen vorzugsweise gleich groß sind.

4. Dilatations-Ballonkatheter nach Anspruch 3, dadurch gekennzeichnet, daß n und m gleich 2 oder 3 sind.

5. Dilatations-Ballonkatheter nach einem der vorhergehenden Ansprüche, dadurch gekennzeichnet, daß mindestens ein Strang, vorzugsweise alle Stränge aus je einem Faden bestehen.

6. Dilatations-Ballonkatheter nach einem der vorhergehenden Ansprüche, dadurch gekennzeichnet, daß mindestens ein Strang, vorzugsweise alle Stränge aus je einem mehrere Fäden aufweisenden Fadenbündel bestehen.

7. Dilatations-Ballonkatheter nach einem der vorhergehenden Ansprüche, dadurch gekennzeichnet, daß die Stränge (23, 23') unter sich gleiche metrische Nummer aufweisen.

8. Dilatations-Ballonkatheter nach einem der vorhergehenden Ansprüche, dadurch gekennzeichnet, daß die elastische Dehnung der Fäden (27, 27') der Fadenscharen (21, 22) mindestens 20% beträgt.

9. Dilatations-Ballonkatheter nach einem der vorhergehenden Ansprüche, dadurch gekennzeichnet, daß die elastische Dehnung der Fäden (27, 27') der Fadenscharen (21, 22) mindestens 50%, vorzugsweise mindestens 100% und/oder maximal 800%, vorzugsweise maximal 500% beträgt.

10. Dilatations-Ballonkatheter nach einem der vorhergehenden Ansprüche, dadurch gekennzeichnet, daß die Reißdehnung der Fäden der Stränge (23, 23') mindestens 1%, vorzugsweise mindestens 2% und/oder maximal 10%, vorzugsweise maximal 6%, insbesondere maximal 5% beträgt.

11. Dilatations-Ballonkatheter nach einem der vorhergehenden Ansprüche, dadurch gekennzeichnet, daß die Katheterleitung (11) ein flexibler Schlauch ist.

12. Dilatations-Ballonkatheter nach einem der Ansprüche 1 bis 11, dadurch gekennzeichnet, daß der Flechtschlauch (15) Durchbrüche aufweist.

13. Dilatations-Ballonkatheter nach Anspruch 12, dadurch gekennzeichnet, daß der Flechtschlauch (15) außenseitig und innenseitig mit je einem fluidumsdichten Mantel (16, 17) versehen ist, welche Mäntel vorzugsweise durch Durchbrüche des Flechtschlauches (15) hindurch stellenweise miteinander verklebt oder verschweißt sind und/oder der äußere Mantel (16) über mindestens ein axiales Ende des Flechtschlauches übersteht.

14. Dilatations-Ballonkatheter nach einem der vorhergehenden Ansprüche, dadurch gekennzeichnet, daß der Flechtschlauch (15) an zumindest einem seiner beiden Längsendbereiche durch Umwickeln mit mindestens einem Faden (24) (nachfolgend Umwicklungsfaden genannt) auf der Katheterleitung (11) fest und über den umwickelten Bereich bzw. über die umwickelten Bereiche unaufweitbar gehalten ist, wobei vorzugsweise vorgesehen ist, daß dieser Flechtschlauch einschließlich seines mindestens einen von mindestens einem Umwicklungsfaden umwickelten Längsbereiches mit einem äußeren, elastischen, fluidumsdichten Mantel (16) versehen ist.

15. Dilatations-Ballonkatheter nach einem der Ansprüche 1 bis 13, dadurch gekennzeichnet, daß die beiden axialen Endbereiche des Flechtschlauches mittels thermoplastischen, durch Wärmeanwendung schrumpfbaren Schrumpfschläuchen auf der Katheterleitung gehalten und der Schrumpfschlauch sowohl mit der Katheterleitung als auch mit dem Flechtschlauch verklebt oder verschweißt ist.

16. Dilatations-Ballonkatheter nach einem der vorhergehenden Ansprüche, dadurch gekennzeichnet, daß die Katheterleitung (11) an einem Ende fluidumsdicht abgeschlossen ist.

17. Dilatations-Ballonkatheter nach einem der vorhergehenden Ansprüche, dadurch gekennzeichnet, daß die Katheterleitung ein einziges Lumen (13) aufweist.

18. Dilatations-Ballonkatheter nach einem der Ansprüche 1 bis 16, dadurch gekennzeichnet, daß die Katheterleitung ein Hauptlumen (13') und mindestens ein Nebenlumen (13'') aufweist, wobei nur das mindestens eine Nebenlumen dem Leiten von dem Aufweiten des Ballons dienenden Druckfluid dient.

19. Dilatations-Ballonkatheter nach einem der vorhergehenden Ansprüche, dadurch gekennzeichnet, daß der Flechtschlauch (15) so ausgebildet ist, daß der lichte Innendurchmesser des Ballons (12), wenn er durch Druckfluid so weit aufgeweitet ist, daß die Stränge (23, 23') auf ca. 90% ihrer Reißdehnung beansprucht sind, im aufgeweiteten Bereich maximal das 5-fache, vorzugsweise maximal das 2-fache, insbesondere maximal das 1,5-fache des lichten Innendurchmessers des nicht aufgeweiteten Ballons beträgt.

20. Dilatations-Ballonkatheter nach einem der vorhergehenden Ansprüche, dadurch gekennzeichnet, daß die Fäden der Stränge (23, 23') aus hochmolekularem Polyethylen und/oder Para-Aramid und/oder Kohlenstoff bestehen.

21. Dilatations-Ballonkatheter nach einem der vorhergehenden Ansprüche, dadurch gekennzeichnet, daß die Fäden der Stränge (23, 23') Monofilamente und/oder Multifilamente sind.

7

22. Dilatations-Ballonkatheter nach einem der vorhergehenden Ansprüche, dadurch gekennzeichnet, daß die Länge des aufweitbaren Bereichs des Ballons (12) mindestens das 5-fache, vorzugsweise mindestens das 15-fache des maximalen Außendurchmessers dieses Ballonbereiches in unaufgeweitetem Zustand beträgt.

23. Dilatations-Ballonkatheter nach eine der vorhergehenden Ansprüche, dadurch gekennzeichnet, daß die Zahl der Stränge (23, 23') des Flechtschlauches (15) maximal der Zahl der Klöppel, vorzugsweise der halben Klöppelzahl, entspricht, von denen die beiden Fadenscharen (21, 22) beim Flechten des Flechtschlauches (15) kamen.

24. Dilatations-Ballonkatheter nach einem der vorhergehenden Ansprüche, dadurch gekennzeichnet, daß der Ballon in nicht aufgeweitetem Zustand, in welchem der aufweitbare Bereich vorzugsweise an der Katheterleitung dicht anliegt, keine Längsfalten aufweist, vorzugsweise völlig faltenfrei ausgebildet ist.

## Claims

1. Dilation balloon-type catheter (10) having an elongate hollow catheter line (11) on which there is arranged axially non-displaceably a flexible balloon (12) which is expandable by pressurized fluid, this fluid being introducible through at least one hole (13) in the catheter line (11) into the intermediate space between the balloon (12) and the catheter line (11) and being removable therefrom again, this balloon (12) having a braided hose (15) which is provided with at least one fluid-tight elastic casing (16, 17), characterized in that the braided hose (15) is braided from two sheaves (21, 22) of threads running in opposite directions and comprising elastomeric threads, and there are braided into this braiding strands (23, 23') of high-modulus fibres running in the axial direction of the braided hose (15) during braiding in such a way that the individual strand runs, as seen from the outside, over the threads of one sheaf of threads (21) and under the threads of the other sheaf of threads (22), and in that the length of the expandable region of the balloon (12) is at least 4 times the maximum external diameter of this balloon region in the unexpanded state.

2. Dilation balloon-type catheter according to Claim 1, characterized in that adjacent strands (23, 23') of the braided hose are laid such that one strand (23) runs, as seen from the outside, over the threads of the first sheaf of threads (21) and runs under the threads of the second sheaf of threads (22), whereas the other strand (23') runs under the threads of the first sheaf of threads (21) and over those of the second sheaf of threads (22).

3. Dilation balloon-type catheter according to Claim 1, characterized in that the strands of the braided hose are laid such that alternate groups comprising n mutually adjacent strands run, as seen from the outside, over the threads of the first sheaf of threads and under the threads of the second sheaf of threads, and in that the intermediate groups of strands, of which each comprises m strands respectively, run under the threads of the first sheaf of threads and over the threads of the second sheaf of threads, n and m each being an integer larger than 1 and n and m preferably being the same for both groups of strands.

4. Dilation balloon-type catheter according to Claim 3, characterized in that n and m are equal to 2 or 3.

5. Dilation balloon-type catheter according to one of the preceding claims, characterized in that at least one strand, preferably all the strands, each comprise one thread.

6. Dilation balloon-type catheter according to one of the preceding claims, characterized in that at least one strand, preferably all the strands, each comprise a bundle of threads having a plurality of threads.

7. Dilation balloon-type catheter according to one of the preceding claims, characterized in that the strands (23, 23') have the same metric count as one another.

8. Dilation balloon-type catheter according to one of the preceding claims, characterized in that the elastic stretch of the threads (27, 27') of the sheaves of threads (21, 22) is at least 20%.

9. Dilation balloon-type catheter according to one of the preceding claims, characterized in that the elastic stretch of the threads (27, 27') of the sheaves of threads (21, 22) is at least 50%, preferably at least 100% and/or at most 800%, preferably at most 500%.

10. Dilation balloon-type catheter according to one of the preceding claims, characterized in that the elongation at tear of the threads of the strands (23, 23') is at least 1%, preferably at least 2% and/or at most 10%, preferably at most 6%, in particular at most 5%.

11. Dilation balloon-type catheter according to one of the preceding claims, characterized in that the catheter line (11) is a flexible hose.

12. Dilation balloon-type catheter according to one of Claims 1 to 11, characterized in that the braided hose (15) has perforations.

13. Dilation balloon-type catheter according to Claim 12, characterized in that the braided hose (15) is provided on the outside and inside with a respective fluid-tight casing (16, 17), these casings preferably being fused or stuck with adhesive to one another at points, preferably through perforations of the braided hose (15), and/or the outer casing (16) projecting over at least one axial end of the braided hose.

14. Dilation balloon-type catheter according to one of the preceding claims, characterized in that the braided hose (15) is held firmly on the catheter line (11) at least one of its two longitudinal end regions by being wound around by at least one thread (24) (called winding thread below), and is kept unexpandable over the wound region or regions, it preferably being provided that this braided hose, including its at least one longitudinal region wound around by at least one winding thread, is provided with an outer elastic fluid-tight casing (16).

15. Dilation balloon-type catheter according to one of Claims 1 to 13, characterized in that the two axial end regions of the braided hose are held on the catheter line by means of thermoplastic shrink hoses which are shrinkable by the application of heat, and the shrink hose is fused or stuck with adhesive both to the catheter line and also to the braided hose.

16. Dilation balloon-type catheter according to one of the preceding claims, characterized in that the catheter line (11) is closed off at one end in fluid-tight manner.

17. Dilation balloon-type catheter according to one of the preceding claims, characterized in that the catheter line has a single hole (13).

18. Dilation balloon-type catheter according to one of Claims 1 to 16, characterized in that the catheter line has a main hole (13') and at least one secondary hole (13"), only the at least one secondary hole serving to guide pressurized fluid serving to expand the balloon.

19. Dilation balloon-type catheter according to one of the preceding claims, characterized in that the braided hose (15) is constructed such that the clear internal diameter of the balloon (12), when it is expanded by pressurized fluid to such an extent that the strands (23, 23') are loaded to approximately 90% of their elongation at tear, is in the expanded region at most 5 times, preferably at most twice, in particular at most 1.5 times the clear internal diameter of the unexpanded balloon.

20. Dilation balloon-type catheter according to one of the preceding claims, characterized in that the threads of the strands (23, 23') comprise high-molecular polyethylene and/or para-aramid and/or carbon.

21. Dilation balloon-type catheter according to one of the preceding claims, characterized in that the threads of the strands (23, 23') are monofilaments and/or multifilaments.

22. Dilation balloon-type catheter according to one of the preceding claims, characterized in that the length of the expandable region of the balloon (12) is at least 5 times, preferably at least 15 times the maximum external diameter of this balloon region in the unexpanded state.

23. Dilation balloon-type catheter according to one of the preceding claims, characterized in that the number of strands (23, 23') of the braided hose (15) corresponds at most to the number of bobbins, preferably half the number of bobbins, from which the two sheaves of threads (21, 22) came during braiding of the braided hose (15).

24. Dilation balloon-type catheter according to one of the preceding claims, characterized in that the balloon has, in the unexpanded state in which the expandable region preferably bears closely against the catheter line, no longitudinal folds, and is preferably constructed to be completely free of folds.

## Revendications

1. Cathéter à ballon de dilatation (10) comportant une conduite de cathéter (11), allongée, creuse portant de manière non glissante axialement, un ballon souple (12) extensible par action d'un fluide comprimé, ce fluide comprimé pouvant être introduit dans l'intervalle entre le ballon (12) et la conduite de cathéter (11) par au moins un passage (13) dans la conduite de cathéter (11) et être de nouveau évacué de cet intervalle, ce ballon (12) ayant un tuyau tressé (15) muni d'au moins une enveloppe (16, 17) élastique étanche au fluide, cathéter caractérisé en ce que le tuyau tressé (15) se compose de deux nappes de fils (21, 22) à sens opposé, formées de fils en élastomère, tressés, et ce tressage intègre dans la direction axiale du tuyau tressé (15), des brins (23, 23') en fibres à module élevé de façon que vu de l'extérieur, un brin passe par-dessus les fils d'une nappe de fils (21) et en dessous des fils de l'autre nappe de fils (22) et en ce que la longueur de la zone extensible du ballon (12) correspond à au moins quatre fois de diamètre extérieur maximum de cette zone de ballon à l'état non gonflé.

2. Cathéter à ballon de dilatation selon la revendication 1, caractérisé en ce que des brins voisins (23, 23') du tuyau tressé sont disposés pour que l'un des brins (23) passe par-dessus les fils de la première nappe de fils (21) lorsqu'on regarde de l'extérieur et en-dessous des fils de la seconde nappe de fils (22) et l'autre brin (23') passe en-dessous des fils de la première nappe de fils (21) et par-dessus ceux de la seconde nappe de fils (22).

3. Cathéter à ballon de dilatation selon la revendication 1, caractérisé en ce que les brins du tuyau tressé sont disposés pour que le second des groupes parmi n brins voisins passe par-dessus les fils de la première nappe de fils lorsqu'on regarde de l'extérieur et en-dessous des fils de la seconde nappe de fils et en ce que les groupes de brins intermédiaires composés chaque fois de m brins, passent sous les fils de la première nappe de fils et par-dessus les fils de la seconde nappe de fils, n et m étant un nombre entier supérieur à 1 et n et m étant de préférence égaux pour les deux groupes de brins.

4. Cathéter à ballon de dilatation selon la revendication 3, caractérisé en ce que n et m sont égaux à 2 ou 3.

5. Cathéter à ballon de dilatation selon l'une des revendications précédentes, caractérisé en ce qu'au moins un brin et de préférence tous les brins sont constitués d'un fil.

6. Cathéter à ballon de dilatation selon l'une des revendications précédentes, caractérisé en ce qu'au moins un brin et de préférence tous les brins se composent chaque fois de faisceaux de fils à plusieurs fils.

7. Cathéter à ballon de dilatation selon l'une des revendications précédentes, caractérisé en ce que les brins (23, 23') ont le même nombre métrique.

8. Cathéter à ballon de dilatation selon l'une des revendications précédentes, caractérisé en ce que l'allongement élastique des fils (27, 27') des nappes de fils (21, 22) est au moins égal à 20%.

9. Cathéter à ballon de dilatation selon l'une des revendications précédentes, caractérisé en ce que l'allongement élastique des fils (27, 27') des nappes de fils (21, 22) est au moins égal à 50%, de préférence au moins égal à 100% et/ou au maximum égal à 800% et de préférence au maximum égal à 500%.

10. Cathéter à ballon de dilatation selon l'une des revendications précédentes, caractérisé en ce que l'allongement à la rupture des fils des brins (23, 23') est au moins égal à 1%, de préférence au moins égal à 2% et/ou au maximum de 10%, de préférence au maximum de 6% notamment au maximum de 5%.

11. Cathéter à ballon de dilatation selon l'une des revendications précédentes, caractérisé en ce que la conduite de cathéter (11) est un tuyau souple.

12. Cathéter à ballon de dilatation selon l'une des revendications 1 à 11, caractérisé en ce que le tuyau tressé (15) comporte des orifices.

13. Cathéter à ballon de dilatation selon la revendication 12, caractérisé en ce que le tuyau tressé (15) est muni sur son côté extérieur et sur son côté intérieur chaque fois d'une enveloppe (16, 17) étanche au fluide, ces enveloppes étant de préférence collées ou soudées l'une à l'autre, par endroit, de préférence à travers des orifices du tuyau tressé (15) et/ou l'enveloppe extérieure (16) déborde d'au moins une extrémité axiale du tuyau tressé.

14. Cathéter à ballon de dilatation selon l'une des revendications précédentes, caractérisé en ce que le tuyau tressé (15) est fixé solidairement sur la conduite de cathéter (11) dans au moins l'une de ses zones d'extrémité longitudinales par enroulement avec au moins un fil (24) (appelé ci-après fil d'enroulement) et est maintenu de manière inextensible dans la zone d'enroulement ou les zones d'enroulement, et il est prévu de préférence que ce tuyau tressé y compris au moins l'une de ses zones longitudinales munies d'un enroulement par fil est garni d'une enveloppe extérieure (16) élastique étanche au fluide.

15. Cathéter à ballon de dilatation selon l'une des revendications 1 à 13, caractérisé en ce que les deux zones d'extrémité axiales du tuyau tressé sont collées ou soudées par des tuyaux thermoplastiques, thermo-rétractables sur la conduite de cathéter et le tuyau rétractable est collé ou soudé à la fois à la conduite de cathéter et au tuyau tressé.

16. Cathéter à ballon de dilatation selon l'une des revendications précédentes, caractérisé en ce que la conduite de cathéter (11) est fermée de manière étanche au fluide à une extrémité.

17. Cathéter à ballon de dilatation selon l'une des revendications précédentes, caractérisé en ce que la conduite de cathéter comporte un seul passage (13).

18. Cathéter à ballon de dilatation selon l'une des revendications 1 à 16, caractérisé en ce que la conduite de cathéter comporte un passage principal (13') et au moins un passage auxiliaire (13''), et seulement un passage auxiliaire sert au passage du fluide comprimé servant à gonfler le ballon.

19. Cathéter à ballon à dilatation selon l'une des revendications précédentes, caractérisé en ce que le tuyau tressé (15) est conçu pour le diamètre intérieur libre du ballon (12) lorsqu'il est gonflé par du fluide comprimé ne se déploie que jusqu'à ce que les brins (23, 23') soient sollicités à environ 90% de leur allongement à la rupture, dans la zone étendue au maximum 5 fois et de préférence au maximum 2 fois et notamment au maximum 1,5 fois le diamètre intérieur libre du ballon non gonflé.

20. Cathéter à ballon de dilatation selon l'une des revendications précédentes, en ce que les fils des brins (23, 23') sont en polyéthylène à poids moléculaire élevé et/ou en para-aramide et/ou en carbone.

21. Cathéter à ballon de dilatation selon l'une des revendications précédentes, caractérisé en ce que les fils des brins (23, 23') sont des monofilaments et/ou des multifilaments.

22. Cathéter à ballon de dilatation selon l'une des revendications précédentes, caractérisé en ce que la longueur de la zone gonflée du ballon (12) correspond au moins 5 fois et de préférence au moins 15 fois le diamètre maximum extérieur de cette zone de ballon à l'état non gonflé.

23. Cathéter à ballon de dilatation selon l'une des revendications précédentes, caractérisé en ce que le nombre des brins (23, 23') du tuyau tressé (15) correspond au maximum au nombre de fuseaux de préférence à la moitié du nombre des fuseaux dont sont dévidées les deux nappes de fils (21, 23) lors du tressage du tuyau (15).

24. Cathéter à ballon de dilatation selon l'une des revendications précédentes, caractérisé en ce qu'à l'état non gonflé, lorsque la zone extensible est appliquée de préférence étroitement contre la conduite de cathéter, le ballon ne présente pas de plis longitudinaux et est de préférence réalisé sans aucun pli.

FIG.1

13

11

12

12

10

15

23'

23

21

23

22

27'

27

23'

23'

23

FIG.6

15

13

15

16

14

17

FIG.3

10

12

11

EP 0 331 040 B1

FIG.2

13

FIG.4

FIG.5